# FASCICULE DE BREVET EUROPEEN

(11) **EP 2 916 965 B1**
(45) Date de publication et mention de la délivrance du brevet: **14.03.2018**
(21) Numéro de dépôt: 13803128.1
(22) Date de dépôt: 08.11.2013
(51) Int. Cl.: B05B 7/24, B05B 7/00, B05B 12/02, B05B 12/08, A01M 29/12, A61M 15/00, A01M 1/20, A61M 11/06, A61M 16/00

(54) **CARTOUCHE A OBSOLESCENCE PROGRAMMEE, DE PRODUCTION ET DE DIFFUSION D'UN AEROSOL, ET APPAREIL DE DIFFUSION LA COMPORTANT**
MIT GESPEICHERTER OBSOLESZENZ AUSGETATTETE KARTUSCHE ZUR ERZEUGUNG UND AUSGABE EINES AEROSOLS UND SPRÜHVORRICHTUNG MIT EINER SOLCHEN KARTUSCHE
PLANNED OBSOLESCENCE CARTRIDGE, FOR PRODUCING AND DISPENSING AN AEROSOL, AND SPRAYING APPARATUS COMPRISING THE SAME

(30) Priorité: 08.11.2012 FR 1260595
(43) Date de publication de la demande: 16.09.2015
(73) Titulaire: Benalikhoudja, Karim, 64170 Serres Sainte Marie (FR)
(72) Inventeur: Benalikhoudja, Karim, 64170 Serres Sainte Marie (FR)
(74) Mandataire: Fragnaud, Aude
(86) Numéro de dépôt international: PCT/FR2013/052697
(87) Numéro de publication internationale: WO 2014/072663

(56) Documents cités:
- EP-A1- 0 799 622
- FR-A1- 2 377 946
- FR-A1- 2 947 191
- US-A- 2 249 359

## Description

### Domaine technique.

La présente invention est du domaine des dispositifs utilisés pour former et diffuser sous forme de micro gouttelettes, une substance liquide dans l'atmosphère d'un local à usage d'habitation ou à usage commercial et concerne plus particulièrement une cartouche de production et de diffusion d'un aérosol et un appareil la comportant, l'aérosol pouvant être formé à partir d'un parfum sous forme liquide, d'un répulsif pour animaux, sous forme liquide, d'un désinfectant sous forme liquide pour la décontamination bactériologique de l'air et des surfaces des locaux, de substances curatives sous forme liquide à visée thérapeutique, par exemple pour le traitement des voies respiratoires des malades en aérosolthérapie, ou bien à visée vétérinaire par exemple pour la vaccination des animaux d'un élevage. L'aérosol peut être formé à partir d'un combustible liquide et d'un gaz comburant en vue de l'alimentation de moteurs thermiques à source d'énergie interne aux cylindres.

### État de la technique antérieure.

On connaît de l'état de la technique divers appareils de nébulisation d'une substance liquide du type précité. Typiquement, ces appareils comprennent un réservoir prévu pour contenir la substance liquide à nébuliser, une buse de nébulisation en relation de communication avec le réservoir, apte à fractionner la substance parfumée en fines gouttelettes et la diffuser sous cette forme dans l'atmosphère et un compresseur d'air actionné par un moteur électrique apte à produire un courant d'air vecteur introduit dans la buse de nébulisation afin que la substance à nébuliser soit d'abord aspirée vers la buse par effet Venturi, ensuite fractionnée dans cette dernière et enfin propulsée vers l'extérieur toujours par le courant d'air porteur.

Un appareil de ce genre est notamment connu du brevet FR 2 947 191 du demandeur. Ce document divulgue une cartouche selon le préambule de la revendication 1. On pourra se reporter au document FR 2 377 946. Ce document, concerne un récipient de conditionnement et de distribution de liquide comportant une pompe manuelle. Comme indiqué dans ce document, la pompe manuelle est fixée de manières amovible, par un pas de vis, au goulot du récipient soit directement soit par l'entremise d'un raccord 8 fixé de manière indémontable au récipient. Dès lors l'objet de ce document ne forme pas un ensemble indémontable. Le raccord 8 a pour objet d'obturer le goulot du récipient et de rendre difficile le remplissage de ce dernier. Cependant ce remplissage peut être tout de même réalisé au travers des orifices 10 et 11 que ce raccord comporte. Le récipient selon ce document peut donc être réutilisé.

### Exposé de l'invention.

### Problème technique.

Pour la plupart des appareils de l'art antérieur, le réservoir est amovible et une fois vide peut aisément être remplacé et rempli. L'inconvénient d'une telle disposition est que le réservoir peut être rechargé en produits liquides incompatibles avec notamment les caractéristiques de la buse qui peut se trouver encrassée ou même fortement endommagée si le produit liquide est corrosif vis-à-vis du matériau qui la compose. En outre, le réservoir, par erreur ou malveillance peut être rechargé en produits liquides incompatibles avec l'utilisation normale de l'appareil, voire en produits toxiques pour les occupants du local ou de l'habitation dans lequel ces produits doivent être diffusés.

### Solution technique.

La présente invention a pour but de pallier les inconvénients précédemment évoqués en écartant tout risque d'erreur de nébulisation et de diffusion d'une substance impropre à l'usage envisagé et en interdisant toute utilisation ultérieure après épuisement du liquide.

Un autre but de la présente invention est une cartouche peu coûteuse à fabriquer et recyclable après usage.

À cet effet la présente invention a pour objet une cartouche de nébulisation selon la revendication 1. Grâce à cette cartouche, on écarte tout risque de diffusion de substances impropres à l'usage envisagé et notamment de substances toxiques ou autres substances non désirées.

Selon une autre caractéristique de l'invention, le compresseur d'air est équipé d'un moteur électrique d'actionnement et ledit compresseur avec son moteur sont disposés dans la cartouche.

Une telle disposition confère à la cartouche des caractéristiques de compacité et d'autonomie en termes de fonctionnement.

Selon une autre caractéristique de l'invention, la cartouche est formée d'une enceinte dont la partie inférieure de son volume interne forme réservoir de liquide et dont la partie supérieure de son volume interne reçoit le compresseur d'air et son moteur ainsi que la buse diphasique, ladite enceinte recevant une paroi de cloisonnement en forme d'entonnoir assurant une séparation entre la partie supérieure et la partie inférieure du volume interne de ladite enceinte. Cette séparation assure une étanchéité permettant au liquide de ne pas couler hors de la cartouche si celle-ci se renverse.

Selon une autre caractéristique de l'invention, le compresseur et son moteur sont disposés dans un fourreau cylindrique s'étendant verticalement dans la partie supérieure du volume interne de l'enceinte et de manière centrée par rapport à cette dernière, ledit fourreau présentant une paroi de fond et étant obturé en partie supérieure par un couvercle pourvu de perçages traversants d'entrée d'air organisés chacun en chicane.

Selon une autre caractéristique de l'invention, le fourreau, en combinaison avec l'enceinte, délimite la chambre de détente de l'aérosol, l'aérosol pénètre dans la chambre de détente par la partie inférieure de cette dernière, le fourreau est réalisé en une matière conductrice de la chaleur afin de réaliser un échange de chaleur avec l'aérosol formé, le fourreau, en partie supérieure, présente une collerette périphérique d'obturation de la partie supérieure du volume interne de l'enceinte et de la chambre de détente, et ladite collerette présente un perçage traversant d'évacuation de l'aérosol.
Grâce à une telle disposition, la paroi du fourreau est balayée par l'aérosol et un échange thermique s'opère entre l'aérosol et ladite paroi. La chaleur dégagée tant par le compresseur que par son moteur se trouve ainsi communiquée à l'aérosol et est évacuée par ce dernier. Une telle disposition est propice à faciliter le refroidissement du compresseur et du moteur associé mais aussi à accélérer l'évaporation des fines gouttelettes de liquide, contenues dans l'aérosol.

Selon une autre caractéristique de l'invention, la sortie d'air comprimé que comporte le compresseur est directement connectée à l'entrée d'air comprimé de la buse diphasique ce qui diminue fortement les pertes de charge.

Une telle disposition est propice d'une part à diminuer la pression d'air nécessaire au bon fonctionnement de la buse diphasique et donc d'utiliser des compresseurs de faible puissance mécanique et par voie de conséquence peu coûteux. En outre, le moteur du compresseur pourra alors être de faible puissance électrique, en rapport avec la puissance du compresseur, ce qui diminuera encore le coût de la cartouche. Ce moteur électrique pourra alors être alimenté avec une tension faible d'environ quatre volts. La pression délivrée par le compresseur sera de l'ordre de 50 millibars.

Selon une autre caractéristique la tête du compresseur pourra avoir directement le profil géométrique de la buse permettant ainsi de réduire le nombre de pièces.

Selon une autre caractéristique la tête du compresseur et son corps de pompe pourront faire partie intégrante de la cartouche permettant ainsi de fixer librement n'importe quel type de moteur électrique.

Selon une autre caractéristique de l'invention, la canule d'aspiration comporte au moins un canal interne d'aspiration, le ou chaque canal interne d'aspiration est équipé en partie inférieure d'un filtre et ladite canule est équipée d'un canal d'évacuation vers le réservoir des particules liquides recueillies par une paroi de cloisonnement, en forme d'entonnoir, laquelle assure une séparation entre la partie supérieure et la partie inférieure, formant réservoir de liquide, du volume interne d'une enceinte formant la cartouche.

Selon une autre caractéristique de l'invention, la canule est équipée en partie inférieure d'un moyen de crépine.

Selon une autre caractéristique de l'invention, le moyen de crépine détermine d'une part une première chambre interne, inférieure en relation de communication avec le réservoir de liquide et avec un canal d'aspiration formé par un tube engagé dans un logement de la canule et d'autre part une seconde chambre interne, supérieure, en relation de communication d'une part avec le conduit d'évacuation et d'autre part avec la première chambre au travers d'un perçage traversant annulaire.

Selon une autre caractéristique de l'invention, la canule entre le logement accueillant le tube d'aspiration et le canal d'évacuation est dotée d'une chambre verticale longiforme obturée de manière étanche en partie supérieure et ouverte en partie inférieure pour être en relation de communication avec la chambre supérieure du moyen de crépine.

Selon une autre caractéristique de l'invention, les moyens d'obsolescence programmée sont constitués par un circuit électronique connecté au moteur du compresseur pour le piloter et l'alimenter en énergie électrique.

Selon une autre caractéristique de l'invention le circuit électronique est apte à mesurer et totaliser la consommation de liquide nébulisé et comparer cette consommation à une valeur de consigne représentative de la quantité de liquide présente dans le réservoir avant premier usage, et interrompre l'alimentation en énergie électrique du moteur du compresseur lorsque la valeur de consigne est atteinte.

Selon une autre caractéristique de l'invention, le circuit électronique intègre au moins :
- un bloc mémoire contenant une donnée relative à la quantité de liquide présente dans le réservoir avant premier usage de l'appareil, une donnée relative à la nature du liquide et une donnée relative à la viscosité du liquide à nébuliser,
- un microcontrôleur apte à calculer le débit instantané de consommation de liquide, et la quantité de liquide consommée, ledit microcontrôleur étant apte de plus à comparer la quantité consommée à la valeur de consigne,
- une interface de puissance au travers de laquelle le moteur du compresseur se trouve alimenté en énergie électrique, ladite interface de puissance étant pilotée par le microcontrôleur pour interrompre l'alimentation du moteur lorsque la valeur de consigne est atteinte.

Le microcontrôleur est apte à communiquer à la carte électronique de pilotage de la machine (carte mère du boîtier) les données présentes dans son bloc mémoire et en particulier une donnée relative à la fin de son temps de fonctionnement consigné.

Cette communication se fait sur les fils d'alimentation selon un protocole spécifique à la cartouche.

Alternativement, selon une autre caractéristique de l'invention, le circuit électronique est apte à totaliser les durées des cycles de fonctionnement du moteur et comparer la valeur obtenue à une valeur de consigne représentative de la durée maximale autorisée de fonctionnement dudit moteur, et interrompre l'alimentation en énergie électrique dudit moteur du compresseur lorsque la valeur de consigne est atteinte, cette valeur de consigne correspond à la durée requise pour le vidage complet du réservoir. La valeur obtenue par la totalisation des durées des cycles de fonctionnement antérieurs est représentative de la quantité de liquide consommée.

Selon une autre caractéristique de l'invention, le circuit électronique intègre au moins :
- un bloc mémoire contenant une donnée relative à la durée maximale d'utilisation du moteur du compresseur,
- un microcontrôleur apte à mesurer et totaliser les durées des cycles de fonctionnement du moteur du compresseur et comparer la valeur de cette mesure à la valeur de consigne,
- une interface de puissance au travers de laquelle le moteur du compresseur se trouve alimenté en énergie électrique, ladite interface de puissance étant pilotée par le microcontrôleur pour interrompre l'alimentation du moteur lorsque la valeur de consigne est atteinte.
- Le microcontrôleur dialogue en temps réel avec la carte-mère et maintien en mémoire le temps d'utilisation de la cartouche ceci est un avantage lorsque l'on change de cartouche en cours d'utilisation.
- Le microcontrôleur pourra aussi délivrer à la carte mère des informations sur la nature du contenu de la cartouche comme par exemple le nom du liquide, ses dates de fabrication et de péremption et autres données.

### Bref exposé des figures et des dessins.

D'autres avantages, buts et caractéristiques de l'invention apparaîtront à la lecture de la description d'une forme préférée de réalisation, donnée à titre d'exemple non limitatif en se référant aux dessins annexés en lesquels :

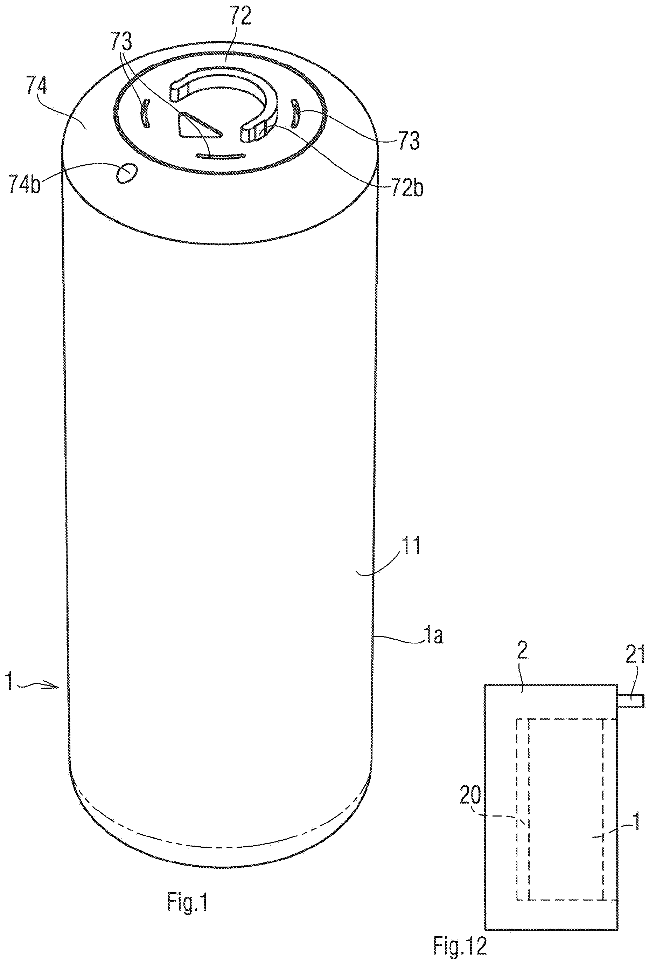

- la figure 1 est une vue en perspective, selon une échelle agrandie, d'une cartouche conforme à l'invention,

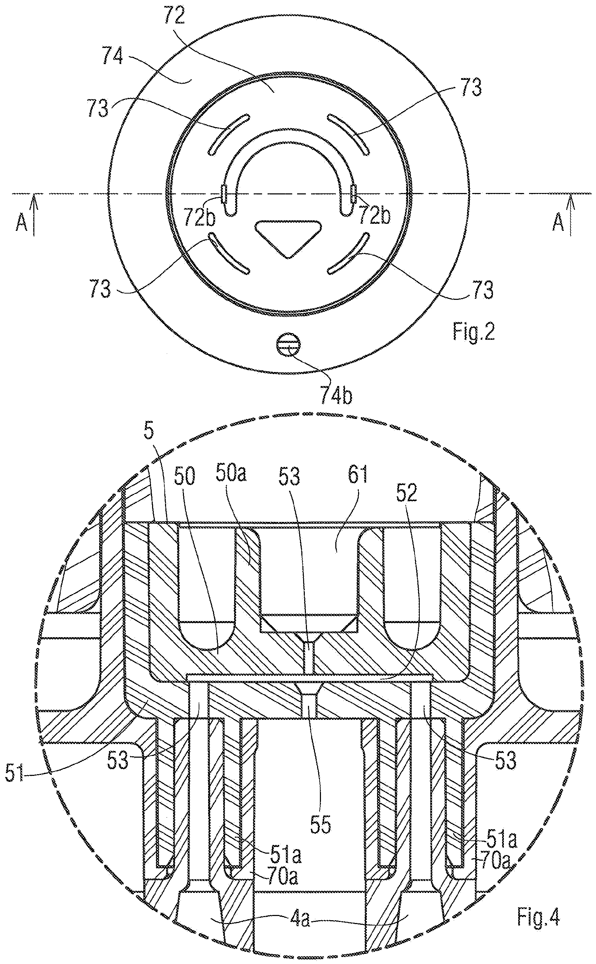

- la figure 2 est une vue de dessus de la cartouche selon l'invention,

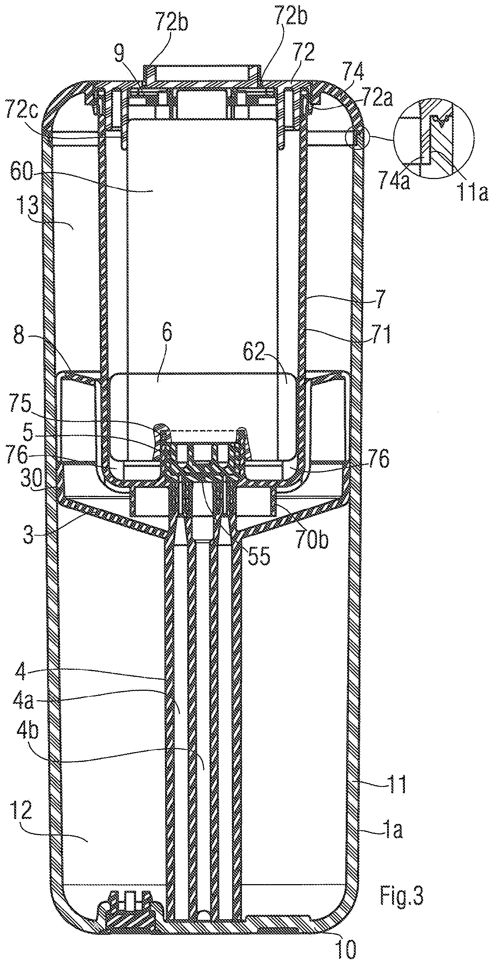

- la figure 3 est une vue en coupe selon la ligne AA de la figure 2,
- la figure 4 est une vue en médaillon montrant le détail de la buse diphasique équipant la cartouche selon l'invention,
- la figure 5 est une vue en perspective d'une paroi de cloisonnement et d'une canule d'aspiration,
- la figure 5a est une vue de face d'une variante de réalisation d'une paroi de cloisonnement avec canule d'aspiration,
- la figure 5b est une vue en coupe d'une autre variante d'exécution d'une canule avec paroi de cloisonnement,
- les figures 5c et 5d sont respectivement des vues en perspective de dessus et de dessous du moyen de crépine,
- la figure 6 est une vue en perspective d'un fourreau,
- la figure 7 est une vue en coupe longitudinale du fourreau selon la figure 6,

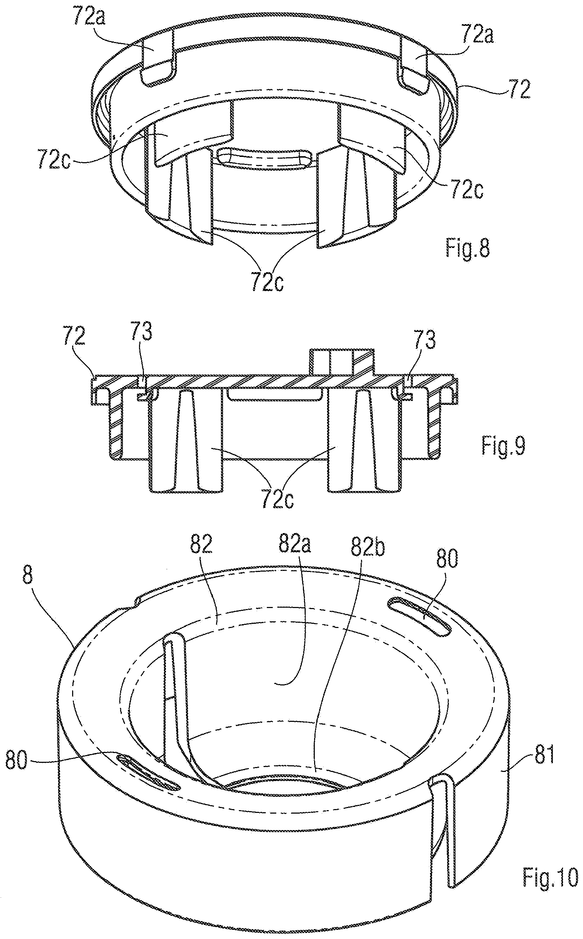

- la figure 8 est une vue en perspective de dessous du couvercle associé au fourreau,
- la figure 9 est une vue en coupe de ce couvercle,
- la figure 10 est une vue en perspective du berceau dans lequel repose le fourreau,

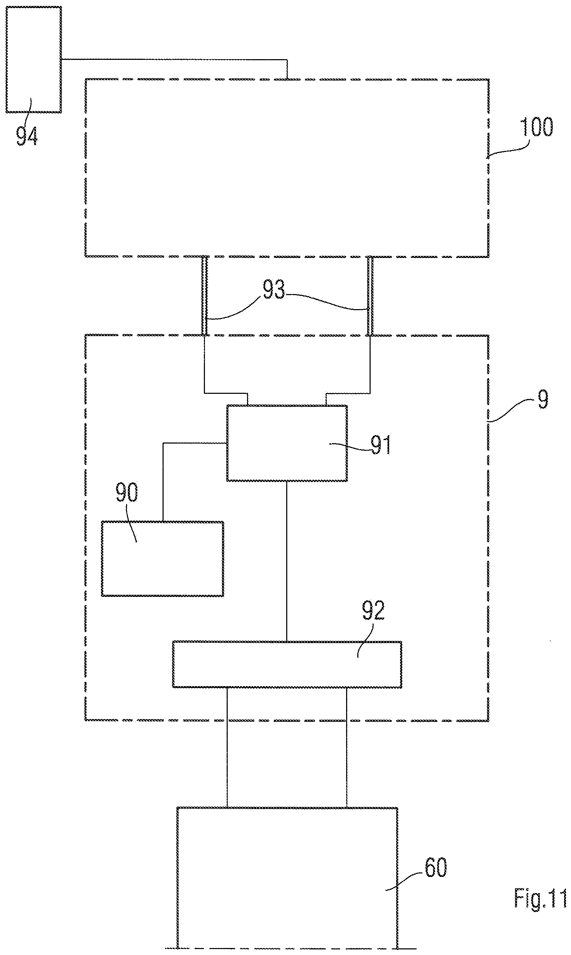

- la figure 11 est une vue schématique du circuit électronique,
- la figure 12 est une vue selon une échelle réduite d'un appareil de diffusion équipé d'une cartouche selon l'invention.

### Meilleure manière de réaliser l'invention.

Telle que représentée, la cartouche 1 selon l'invention, pour la production et diffusion d'un aérosol, est prévue pour être introduite dans un appareil de diffusion d'aérosol 2.

La cartouche 1, selon l'invention, comprend une enceinte 1a close, de forme cylindrique, comportant un fond 10 circulaire et une paroi enveloppe 11 enracinée à ce fond 10.

La partie inférieure du volume interne de l'enceinte 1a forme un réservoir 12 prévu pour recevoir un liquide à nébuliser et est séparée de la partie supérieure du volume interne par une paroi de cloisonnement 3, formant entonnoir autour d'une canule d'aspiration 4 plongeant dans le réservoir 12 et prévue pour alimenter en liquide, une buse diphasique 5 de production d'un aérosol, montée dans la partie supérieure du volume interne de l'enceinte 1a au-dessus de la paroi de cloisonnement 3 et alimentée en air comprimé par un compresseur 6, motorisé monté également avec son moteur 60 dans la partie supérieure du volume interne de l'enceinte 1a.

Le réservoir 12 peut présenter diverses capacités. Ainsi ce dernier, à titre d'exemple non limitatif, pourra présenter des capacités de 20 ml, 200 ml, 5000 ml ou plus.

Selon la forme préférée de réalisation, la paroi de cloisonnement 3 est dotée d'un rebord annulaire 30, cylindrique, orienté vers le haut dont le diamètre externe est égal au diamètre interne de l'enceinte 1a. Par ce rebord annulaire 30, la paroi de séparation 3 est rigidement fixée, par soudage à ultra sons par exemple, à la paroi enveloppe 11 de l'enceinte 1a.

Avantageusement, le compresseur 6 et son moteur 60 ainsi que la buse diphasique 5 sont disposés dans un fourreau cylindrique 7 s'étendant verticalement dans la partie supérieure du volume interne de l'enceinte 1a et de manière centrée par rapport à cette dernière.

Ce fourreau 7 présente une paroi de fond 70 et une paroi enveloppe 71. Ce fourreau 7 est obturé en partie supérieure par un couvercle 72 pourvu de perçages traversants d'entrée d'air 73, organisés chacun en chicane pour rendre difficile l'introduction de corps étranger dans le fourreau 7.

Selon une forme pratique de réalisation, le couvercle 72 est doté de pattes saillantes 72a prévues pour être clipsées dans des empreintes en creux 71a formées dans la paroi enveloppe 71 du fourreau 7, en partie supérieure. Ce couvercle 72 présente des pattes verticales 72c déterminant un logement de calage dans lequel est introduite la partie supérieure du moteur 60 du compresseur 6. En partie inférieure, le fourreau 7, dans son volume interne, présente plusieurs paires d'ailettes radiales 76 déterminant chacune un logement de calage prévu pour recevoir une ailette radiale de calage 62 portée par le compresseur 6. De cette manière l'ensemble compresseur et moteur se trouve parfaitement positionné et maintenu dans le fourreau 7.

De préférence, le fourreau 7, en partie supérieure, présente une collerette périphérique 74 d'obturation de la partie supérieure de l'enceinte 1a. Cette collerette 74 présente un retreint annulaire cylindrique 74a prévu pour venir se loger dans et contre une forme de lamage 11a formée en extrémité supérieure de la paroi enveloppe 11 et y être fixé de manière étanche, par exemple par soudage à ultrasons.

Le fourreau 7, intérieurement sur sa paroi de fond 70, présente un logement 75 dans lequel est montée la buse diphasique 5.

La buse 5 est de préférence conforme à celle décrite dans la demande de brevet FR 2 947 191 et est formée d'un corps de buse en deux parties supérieure 50 et inférieure 51, jointes de manière étanche l'une à l'autre et délimitant toutes deux une chambre de dépression 52 dans laquelle est aspiré, par effet Venturi, le liquide à nébuliser. Plus précisément, la partie supérieure 50 de corps de buse est dotée d'un perçage traversant 53 d'introduction d'air comprimé, en communication avec le volume interne d'un embout de raccordement 50a de la partie 50, dans lequel est engagé de manière étanche, un embout 61 de délivrance d'air comprimé que comporte le compresseur 6. Alternativement, la partie supérieure 50 est intégrée au compresseur 6 et fait corps avec ce dernier.

La partie inférieure 51 de corps de buse comporte au moins un premier perçage traversant 54 d'amené du liquide dans la chambre de dépression 52, ce premier perçage traversant débouchant dans un embout 51a vertical de raccordement que comporte la partie inférieure 51 du corps de buse. Cet embout 51a est engagé dans un perçage traversant pratiqué dans la paroi de fond 70 du fourreau 7 et reçoit la canule d'aspiration 4 laquelle est dotée d'au moins un canal interne d'aspiration 4a venant en relation de communication avec le perçage d'aspiration 54 que comporte la partie inférieure 51 de corps de buse.

Selon une forme pratique de réalisation, l'embout 51a est engagé dans une forme tubulaire 70a, verticale, enracinée à la paroi de fond 70 et formant saillie sous cette dernière.

La partie inférieure 51 du corps de buse, dans l'axe du perçage traversant 53 que comporte la partie supérieure 50, est dotée d'un perçage traversant 55 d'évacuation de l'aérosol, ce dit perçage étant axialement aligné avec un perçage traversant pratiqué dans la paroi de fond 70 du fourreau 7.

De préférence, la partie inférieure 51 de corps de buse présente deux perçages d'aspiration 54 disposés de manière symétrique par rapport au perçage d'évacuation de l'aérosol, tous les deux étant relation de communication respectivement avec deux embouts 51a engagés respectivement dans deux formes tubulaires 70a. Avec une telle forme de réalisation, la canule d'aspiration 4 comporte deux conduits d'aspiration 4a en relation de communication respectivement avec les deux perçages 54 et son extrémité supérieure forme une fourche pour pouvoir être raccordée aux deux embouts 51a.

De façon à réduire le niveau sonore de la buse, la paroi de fond 70, en saillie sous sa face externe au fourreau, pourra présenter une couronne circulaire 70b, se développant autour et à distance de la canule 4 et du perçage 55 d'évacuation de l'aérosol. On peut remarquer que cette couronne pénètre dans la forme d'entonnoir que présente la paroi de cloisonnement 3 et se situe à faible distance de la face supérieure de cette dernière. Cette couronne permet de mieux filtrer l'aérosol en piégeant les grosses particules. Le volume interne que forme cette couronne est de préférence centré par rapport au perçage 55.

La canule d'aspiration 4 traverse de part en part la paroi de cloisonnement 3 et est solidarisée de manière étanche à cette dernière. De préférence, la canule fait corps avec la paroi de cloisonnement 3.

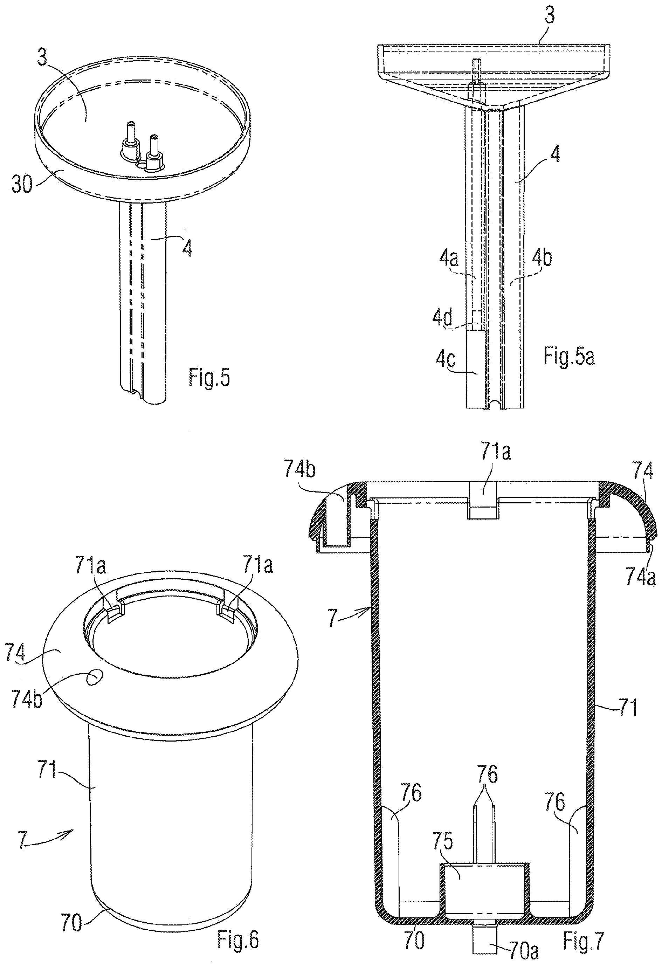

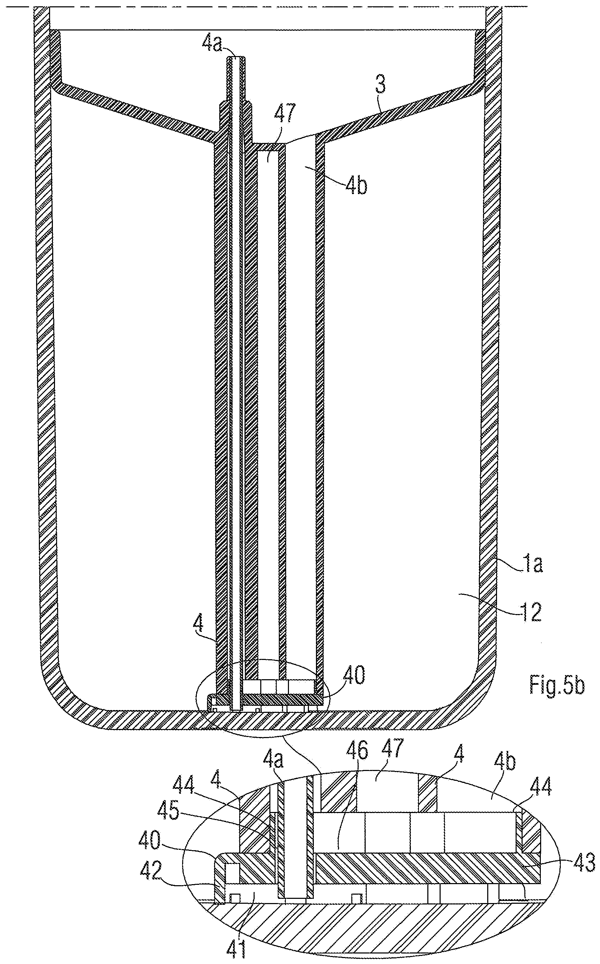

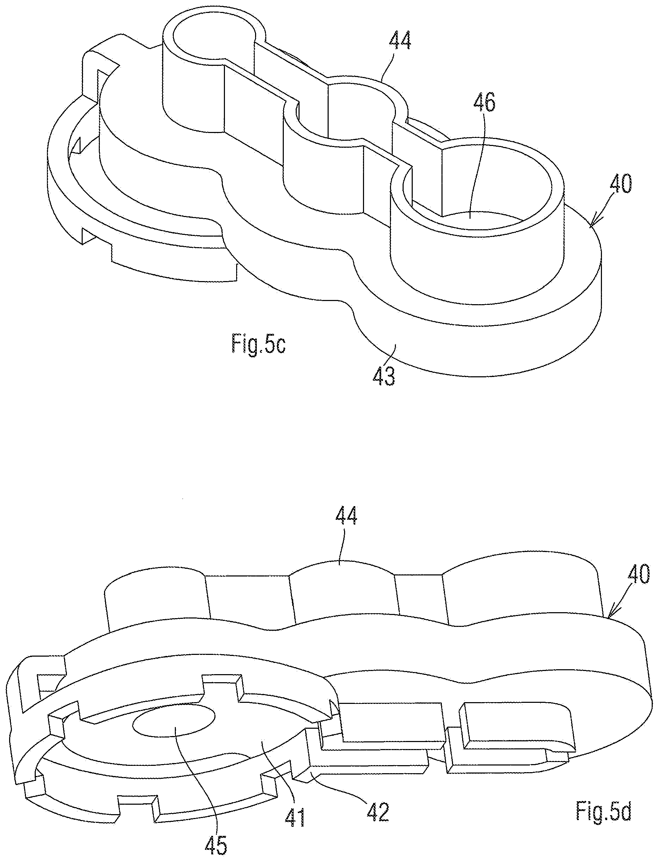

On peut observer que la canule selon la figure 5 comporte deux canaux d'aspiration, tandis que la canule selon les figures 5a et 5b n'en comporte qu'un seul. Le fait de n'utiliser qu'une seule canule permet de limiter le débit de liquide aspiré et de réduire le risque de cavitation de la buse. Le canal d'aspiration 4a pourra recevoir en partie inférieure un filtre 4c, rigide, (Fig 5a) apte à retenir les impuretés et salissures solides que pourrait contenir le liquide. Ce filtre a également pour effet de réguler l'aspiration. Ce filtre 4c est doté d'un manchon de raccordement 4d prévu pour être engagé dans le canal interne 4a de la canule 4.

Le fourreau 7 est réalisé en une matière apte à conduire la chaleur. Ce fourreau 7, par sa partie inférieure, est monté dans un berceau 8 porté par la paroi de séparation 3. Ce berceau 8 assure le maintien du fourreau 7 à distance et au-dessus de la de la paroi de séparation 3. Ainsi l'aérosol délivré par la buse 5 est d'abord introduit dans la région comprise entre le berceau 8 et la paroi de séparation 3 et est ensuite évacué, par passage au travers de perçages traversants 80 pratiqués dans le berceau 8, vers une chambre de détente annulaire 13, formée entre la partie supérieure de l'enceinte et la paroi enveloppe 71 du fourreau 7. Cette chambre de détente 13 est également délimitée par le berceau 8 en partie inférieure et par la collerette 74 en partie supérieure.

Pour permettre l'évacuation de l'aérosol vers l'extérieur depuis cette chambre de détente 13, la collerette 74 est dotée d'un perçage traversant 74b.

Grâce aux dispositions qui viennent d'être décrites, l'aérosol se trouve au contact de la face externe du fourreau 7 et la chaleur dégagée tant par le moteur 60 que par le compresseur 6 se trouve ainsi évacuée par l'aérosol. Une telle disposition assure un refroidissement correct du moteur 60 et du compresseur 6 et favorise l'évaporation des gouttelettes de liquide contenues dans l'aérosol. L'étanchéité du fourreau 7 interdit tout contact entre l'aérosol et le compresseur 6 et son moteur 60.

Grâce au berceau 8 et à la collerette 74, le fourreau 7 est fermement maintenu fixe dans l'enceinte 1a.

Le berceau 8, selon une forme préférée de réalisation, comprend une paroi tubulaire 81 par la bordure inférieure de laquelle il prend appui sur la bordure supérieure du rebord annulaire 30 de la paroi de séparation 3. Le berceau 8 comprend en outre au moins deux pattes 82 de maintien du fourreau 7, enracinées par une aile horizontale qu'elles présentent chacune, à la bordure supérieure de la paroi tubulaire 81. Ces pattes sont angulairement écartées les unes des autres de manière régulière. Les perçages traversant 80 sont formés dans les ailes horizontales des pattes 82. Chaque patte de maintien 82 présente un segment vertical de patte 82a prolongé en partie inférieure par un segment de patte horizontal 82b d'appui du fourreau 7, ce segment se développant de manière radiale vers le centre du berceau formé.

En sortie de buse 5, l'aérosol est orienté verticalement vers le bas et vers la paroi de séparation 3. Les grosses particules de liquide que peut transporter l'aérosol sont d'un poids trop élevé pour être transportées vers la chambre de détente 13 et se déposent sur la paroi de cloisonnement 3. Pour éviter tout engorgement à ce niveau, la canule d'aspiration 4, dans le prolongement d'un perçage traversant axial formé dans la paroi 3, présente un conduit 4b d'évacuation par gravité de ces particules vers le réservoir 12 de liquide. On peut remarquer que le conduit d'évacuation 4b et le ou les conduits d'aspiration 4a que comporte la canule 4 débouchent, par leur extrémité inférieure, au niveau de l'extrémité inférieure de la canule, laquelle extrémité est en appui ou à très faible distance du fond du réservoir 12. Une telle disposition évite que le renversement de la capsule conduise au refluement du liquide vers la partie supérieure de la cartouche 1. La paroi de cloisonnement 3 s'oppose aussi à ce refluement en cas de renversement de la cartouche 1.

En figure 5b est représentée une autre forme de réalisation de la canule 4. Cette canule 4 fait corps avec la paroi de séparation 3 laquelle est toujours sous forme d'entonnoir. Cette canule ne comporte qu'un seul canal d'aspiration 4a formé par un tube et un canal d'évacuation 4b. Le tube formant le canal d'aspiration 4a est introduit dans un logement tubulaire, cylindrique, vertical de la canule 4. En partie supérieure, ce logement cylindrique comporte un rétrécissement pour recevoir, sans jeu fonctionnel, la partie supérieure du tube d'aspiration 4a. Un joint d'étanchéité pourra être disposé, en partie supérieure, entre le logement que comporte la canule 4 et ledit tube. En partie inférieure, un jeu annulaire est ménagé entre le tube et son logement.

Les parties inférieures du tube et de la canule 4 sont préférentiellement engagées dans un moyen de crépine 40 fixé à la paroi de fond du réservoir 12. Ce moyen de crépine 40 détermine au moins une première chambre interne, inférieure 41, en relation de communication avec le volume interne du réservoir 12. Cette chambre est délimitée par une paroi enveloppe 42 et par une paroi supérieure horizontale 43 enracinée à la paroi enveloppe 42. Ce moyen de crépine 40 repose sur le fond du réservoir 12 par la bordure inférieure de la paroi enveloppe 42. La chambre interne inférieure 41 est en relation de communication avec le volume interne du réservoir 12 par des perçages traversants pratiqués notamment dans la paroi enveloppe 42.

La paroi supérieure 43 du moyen de crépine 40 comporte au moins un embout vertical 44 de raccordement, prolongeant un perçage traversant 45 pratiqué dans la paroi supérieure 43 et débouchant dans la première chambre interne inférieure 41. L'embout de raccordement 44 est prévu pour recevoir en emmanchement la partie inférieure de la canule 4. Le tube d'aspiration s'engage dans l'embout 44 et dans ce perçage traversant 45 et par sa partie inférieure est logé dans la chambre inférieure 41. La partie inférieure du tube d'aspiration 4a est disposée à faible distance du fond du réservoir 12. Avantageusement, la chambre inférieure 41 est équipée d'un filtre, non représenté. Ce filtre est apte à retenir les impuretés et salissures solides que pourrait contenir le liquide à diffuser. Ce filtre a également pour effet de réguler l'aspiration.

L'embout de raccordement 44, en combinaison avec la paroi supérieure 43, détermine une seconde chambre interne, supérieure 46. Cette chambre supérieure est en relation de communication d'une part avec le canal d'évacuation 4b de la canule et d'autre part avec la chambre inférieure 41 au travers d'un intervalle annulaire ménagé entre la perçage 45 et le tube d'aspiration 4a. De cette manière, les écoulements recueillis en partie supérieure par l'entonnoir que forme la paroi de séparation 3 peuvent s'écouler vers la première chambre 41 et être repris par le flux de liquide aspiré. De plus la faible largeur de cet intervalle annulaire limite les remontées de liquide vers l'entonnoir 3 lorsque la cartouche, par mégarde, est couchée. Avantageusement entre le conduit d'évacuation 4b et le logement prévu pour accueillir le tube d'aspiration 4a, la canule comporte une chambre verticale longiforme 47 obturée de manière étanche en partie supérieure et ouverte en partie inférieure pour être en relation de communication avec la chambre supérieure 46. Une telle chambre 47 est apte à recueillir les remontées de liquide lorsque la cartouche est couchée. Ainsi cette chambre est apte à limiter les remontées de liquide vers l'entonnoir que forme la paroi 3 et par voie de conséquence, les remontées de liquide vers la partie supérieure de la cartouche. Avantageusement, le conduit d'évacuation 4b est décalé latéralement par rapport au centre de l'entonnoir que forme la paroi de cloisonnement 3.

La cartouche 1 telle que décrite est dotée d'un circuit électronique 9 apte à alimenter et piloter le moteur 60 du compresseur 6.

Selon une première forme de réalisation, le circuit électronique 9 est apte à mesurer et totaliser la consommation de liquide nébulisé et à comparer cette consommation à une valeur de consigne représentative de la quantité de liquide présente dans le réservoir 12 avant premier usage, et interrompre l'alimentation en énergie électrique du moteur 60 du compresseur 6 lorsque la valeur de consigne est atteinte.

Selon cette forme de réalisation, le circuit électronique 9 intègre au moins :
- un bloc mémoire 90 contenant une donnée relative à la quantité de liquide présente dans le réservoir avant premier usage de l'appareil, une donnée relative à la nature du liquide et une donnée relative à la viscosité du liquide à nébuliser,
- un microcontrôleur 91 apte à calculer le débit instantané de consommation de liquide, et la quantité de liquide consommée, ledit microcontrôleur étant apte de plus à comparer la quantité consommée à la valeur de consigne,
- une interface de puissance 92 au travers de laquelle le moteur 60 du compresseur se trouve alimenté en énergie électrique, ladite interface de puissance 92 étant pilotée par le microcontrôleur 91 pour interrompre l'alimentation du moteur 60 lorsque la valeur de consigne est atteinte.

Selon une seconde forme de réalisation, le circuit électronique 9 est apte à mesurer et à totaliser les durées des cycles de fonctionnement du moteur 60 et à comparer la valeur obtenue à une valeur de consigne représentative de la durée maximale autorisée de fonctionnement dudit moteur, et interrompre l'alimentation en énergie électrique dudit moteur 60 lorsque la valeur de consigne est atteinte, cette valeur de consigne correspondant à la durée requise pour le vidage complet du réservoir 12.

Selon cette seconde forme de réalisation de l'invention, le circuit électronique intègre au moins :
- un bloc mémoire 90 contenant une donnée relative à la durée maximale d'utilisation du moteur du compresseur,
- un microcontrôleur 91 apte à mesurer et totaliser les durées des cycles de fonctionnement du moteur du compresseur et comparer la valeur de cette mesure à la valeur de consigne,
- une interface de puissance 92 au travers de laquelle le moteur du compresseur se trouve alimenté en énergie électrique, ladite interface de puissance étant pilotée par le microcontrôleur pour interrompre l'alimentation du moteur lorsque la valeur de consigne est atteinte.

Dans la forme pratique de réalisation, le circuit électronique 9 est monté en totalité ou en partie dans le fourreau 7 entre le moteur 60 du compresseur 6 et le couvercle 72. Ce couvercle 72 présente des perçages traversants 72b de passage de broches électriques 93 d'alimentation en énergie électrique tant du circuit électronique que du moteur 60. Ces broches électriques 93 sont connectées à une source d'énergie électrique 94 externe à la cartouche.

La cartouche 1, telle que décrite, peut être utilisée telle qu'elle ou bien être disposée dans un appareil de diffusion 2 d'un aérosol. Cet appareil 2 se présente de préférence sous la forme d'un boîtier doté d'une chambre interne 20 dans laquelle est disposée de manière amovible la cartouche 1 selon l'invention.

Cet appareil 2 est doté d'un ajutage 21 de délivrance de l'aérosol en regard duquel est positionné le perçage 74b que comporte la cartouche 1. Ce boîtier 2 est équipé des broches 93 et de la source d'énergie électrique 94, cette dernière se présentant sous la forme d'accumulateurs d'énergie électrique basse tension. Alternativement, les broches 93 sont portées par la cartouche et sont prévues pour être connectées à des contacts électriques portés par l'appareil 2.

Cet appareil 2 est également équipé d'un circuit électronique 100 au travers duquel la source d'énergie électrique 94 alimente les broches 93. Le circuit 9 et le circuit électronique 100 sont aptes à échanger des données par l'intermédiaire des broches 93.

Les données échangées pourront être relatives à un identifiant propre à la cartouche 1 et inscrit dans le bloc mémoire 90 du circuit 9 et au temps d'utilisation de la cartouche. Ces données seront inscrites dans un bloc mémoire du circuit 100. Ces dispositions permettront de gérer le changement de cartouche avant épuisement du liquide en conservant dans un bloc mémoire du circuit 100 de l'appareil 2 le temps d'utilisation de chaque cartouche.

L'identifiant de la cartouche permettra à l'appareil de déterminer si cette dernière est conforme et si elle peut être utilisée. Si tel n'est pas le cas, l'alimentation en énergie électrique du moteur 60 sera interdite par le circuit 100.

Le bloc mémoire 90 pourra aussi contenir des données relatives à la nature du contenu de la cartouche comme par exemple la nature du liquide, ses dates de fabrication et de péremption ainsi que d'autres données.

Ces diverses données seront communiquées au circuit 100 et seront inscrites dans un bloc mémoire de ce dernier.

Grâce à ces diverses dispositions, un même appareil 2 pourra gérer plusieurs cartouches 1, lesquelles pourront être utilisées alternativement au gré de l'utilisateur jusqu'à épuisement du liquide qu'elles contiennent.

Il va de soi que la présente invention peut recevoir tous aménagements et variantes du domaine des équivalents techniques sans pour autant sortir du cadre du présent brevet tel que défini par les revendications ci-après.

## Revendications

1. Cartouche de production et de diffusion d'un aérosol (1) comprenant un réservoir (12) contenant un liquide à nébuliser, une buse diphasique de nébulisation (5) en relation de communication avec le réservoir (12) par un canal interne (4a) d'une canule d'aspiration (4) et un compresseur d'air (6) apte à produire un courant d'air comprimé porteur introduit dans la buse diphasique (5) afin que le contenu du réservoir y soit d'abord aspiré par effet Venturi, ensuite fractionné et enfin propulsé d'abord vers une chambre de détente (13) et ensuite vers l'extérieur toujours par le courant d'air porteur, **caractérisée en ce que** les éléments la constituant forment un ensemble indémontable et qu'elle comporte des moyens d'obsolescence programmée aptes à déterminer directement ou indirectement la quantité totale de liquide consommée lors des cycles de fonctionnement antérieurs, aptes à comparer cette valeur à une valeur de consigne représentative de la quantité de liquide présente dans le réservoir avant première utilisation, et aptes à interdire le fonctionnement ultérieur du compresseur dès épuisement du liquide contenu dans le réservoir (12) afin de rendre inutilisable ladite cartouche même si le réservoir de cette dernière est à nouveau rempli.

2. Cartouche selon la revendication 1, **caractérisée en ce que** le compresseur d'air (6) est équipé d'un moteur électrique (60) d'actionnement et que ledit compresseur avec son moteur sont disposés dans ladite cartouche.

3. Cartouche selon la revendication 1 ou la revendication 2, **caractérisée en ce qu'**elle est formée d'une enceinte (1a) dont la partie inférieure de son volume interne forme réservoir (12) de liquide et dont la partie supérieure de son volume interne reçoit le compresseur d'air (6) et son moteur (60) ainsi que la buse diphasique (5) de nébulisation, ladite enceinte (1a) recevant une paroi (3) de cloisonnement en forme d'entonnoir laquelle assure une séparation entre la partie supérieure et la partie inférieure du volume interne de ladite enceinte (1a).

4. Cartouche selon la revendication précédente, **caractérisée en ce que** le compresseur (6) et son moteur (60) sont disposés dans un fourreau (7) cylindrique s'étendant verticalement dans la partie supérieure du volume interne de l'enceinte (1a) et de manière centrée par rapport à cette dernière, ledit fourreau (7) présentant une paroi de fond (70) et étant obturé en partie supérieure par un couvercle (72) pourvu de perçages traversants (73) d'entrée d'air organisés chacun en chicane.

5. Cartouche selon la revendication précédente, **caractérisée en ce que** le fourreau (7) en combinaison avec l'enceinte (1a) délimite la chambre de détente (13) de l'aérosol, que l'aérosol pénètre dans la chambre de détente (13) par la partie inférieure de cette dernière, que le fourreau (7) est réalisé en une matière conductrice de la chaleur afin de réaliser un échange de chaleur avec l'aérosol formé, que le fourreau (7), en partie supérieure, présente une collerette périphérique (74) d'obturation de la partie supérieure du volume interne de l'enceinte (1a) et de la chambre de détente (13), et que ladite collerette (74) présente un perçage traversant (74b) d'évacuation de l'aérosol.

6. Cartouche selon la revendication 4 ou la revendication 5, **caractérisée en ce que** le fourreau (7), par sa partie inférieure est monté dans un berceau (8) porté par la paroi de séparation (3), ledit berceau étant équipé de perçages traversants (80) de passage de l'aérosol vers la chambre de détente (13), ledit fourreau (7) présentant intérieurement sur sa paroi de fond (70), un logement (70a) dans lequel est montée la buse diphasique (5) ; la paroi de fond (70) du fourreau (7) étant dotée d'un perçage traversant axialement aligné avec le perçage (55) d'évacuation d'aérosol de la buse diphasique (5) ; la buse (5) étant dotée d'un perçage d'entrée d'air comprimé (53) en relation de communication avec la sortie d'air comprimé du compresseur (6) et d'au moins un perçage d'aspiration (54) de liquide, en relation de communication par l'intermédiaire du canal interne d'aspiration (4a) de la canule d'aspiration (4), avec le réservoir de liquide (12).

7. Cartouche selon l'une quelconque des revendications précédentes, **caractérisée en ce que** la sortie d'air comprimé que comporte le compresseur (6) est directement connectée à l'entrée d'air comprimé que comporte la buse diphasique (5).

8. Cartouche selon l'une quelconque des revendications 1 à 7, **caractérisée en ce que** les moyens d'obsolescence programmée sont constitués par un circuit électronique (9) connecté au moteur (60) du compresseur pour le piloter et l'alimenter en énergie électrique.

9. Cartouche selon la revendication précédente, **caractérisé en ce que** le circuit électronique (9) est monté dans le fourreau (7) entre le moteur (60) du compresseur (6) et le couvercle (72), que ce couvercle (72) présente des perçages traversants (72b) de passage de broches électriques (93) d'alimentation en énergie électrique tant du circuit électronique (9) que du moteur (60) et que ces broches électriques (93) sont connectées à une source d'énergie électrique (94) externe à ladite cartouche.

10. Cartouche selon la revendication 8 ou la revendication 9, **caractérisée en ce que** le circuit électronique (9) est apte à mesurer et totaliser la consommation de liquide nébulisé et à comparer cette consommation à une valeur de consigne représentative de la quantité de liquide présente dans le réservoir (12) avant premier usage, et interrompre l'alimentation en énergie électrique du moteur (60) du compresseur (6) lorsque la valeur de consigne est atteinte.

11. Cartouche selon la revendication précédente, **caractérisée en ce que** le circuit électronique (9) intègre au moins :
- un bloc mémoire (90) contenant une donnée relative à la quantité de liquide présente dans le réservoir avant premier usage de l'appareil, une donnée relative à la nature du liquide et une donnée relative à la viscosité du liquide à nébuliser,
- un microcontrôleur (91) apte à calculer le débit instantané de consommation de liquide, et la quantité de liquide consommée, ledit microcontrôleur étant apte de plus à comparer la quantité consommée à la valeur de consigne,
- une interface de puissance (92) au travers de laquelle le moteur (60) du compresseur se trouve alimenté en énergie électrique, ladite interface de puissance (92) étant pilotée par le microcontrôleur (91) pour interrompre l'alimentation du moteur (60) lorsque la valeur de consigne est atteinte.

12. Cartouche selon la revendication 8 ou la revendication 9, **caractérisée en ce que** le circuit électronique (9) est apte mesurer et à totaliser les durées des cycles de fonctionnement du moteur (60) et à comparer la valeur obtenue à une valeur de consigne représentative de la durée maximale autorisée de fonctionnement dudit moteur, et interrompre l'alimentation en énergie électrique dudit moteur (60) lorsque la valeur de consigne est atteinte, cette valeur de consigne correspondant à la durée requise pour le vidage complet du réservoir (12).

13. Cartouche selon la revendication précédente, **caractérisée en ce que** le circuit électronique (9) intègre au moins :
- un bloc mémoire (90) contenant une donnée relative à la durée maximale d'utilisation du moteur du compresseur,
- un microcontrôleur (91) apte à mesurer et totaliser les durées des cycles de fonctionnement du moteur du compresseur et comparer la valeur de cette mesure à la valeur de consigne,
- une interface de puissance (92) au travers de laquelle le moteur du compresseur se trouve alimenté en énergie électrique, ladite interface de puissance étant pilotée par le microcontrôleur pour interrompre l'alimentation du moteur lorsque la valeur de consigne est atteinte.

14. Appareil de diffusion d'un aérosol **caractérisé en ce qu'**il reçoit une cartouche selon l'une quelconque des revendications précédentes.

15. Appareil de diffusion d'un aérosol recevant une cartouche selon les revendications 5 et 9 prises ensemble,
**caractérisé en ce qu'**il est doté d'un ajutage (21) de délivrance de l'aérosol en regard duquel est positionné le perçage (74b) que comporte la cartouche (1) et d'un boitier (2) doté d'une chambre interne (20) dans laquelle est disposée de manière amovible la cartouche, ledit boitier étant équipé des broches (93) et de la source d'énergie électrique (94).

## Patentansprüche

1. Patrone für die Herstellung und Abgabe eines Aerosols (1) umfassend einen Behälter (12), der eine zu zerstäubende Flüssigkeit enthält, eine Zweiphasen-Zerstäuberdüse (5), die mit dem Behälter (12) über einen inneren Kanal (4a) einer Saugkanüle (4) in Verbindung steht, und einen Luftkompressor (6), der geeignet ist, einen tragfähigen Druckluftstrom zu erzeugen, der in die Zweiphasen-Düse (5) eingeleitet wird, so dass der Inhalt des Behälters dort zunächst mittels des Venturieffekts angesaugt, dann fraktioniert und schließlich zuerst in eine Expansionskammer (13) und dann durch den Trägerluftstrom nach außen getrieben wird, **dadurch gekennzeichnet, dass** die sie bildenden Bestandteile eine unzerlegbare Baueinheit bilden, und dass sie programmierte Veralterungsmittel umfassen, die geeignet sind, direkt oder indirekt die Gesamtmenge der bei früheren Betriebszyklen verbrauchten Flüssigkeit zu bestimmen, diesen Wert mit einem Sollwert zu vergleichen, der für die vor dem ersten Gebrauch in dem Behälter vorhandene Flüssigkeitsmenge repräsentativ ist, und die geeignet sind, den späteren Betrieb des Kompressors sofort nach Erschöpfung der im Behälter (12) vorhandenen Flüssigkeit zu verbieten, um die Patrone unverwendbar zu machen, und dies sogar, wenn Letzterer erneut befüllt ist.

2. Patrone nach Anspruch 1, **dadurch gekennzeichnet, dass** der Luftkompressor (6) mit einem elektrischen Betätigungsmotor (60) ausgestattet ist, und dass der Kompressor mit seinem Motor in der Patrone angeordnet ist.

3. Patrone nach Anspruch 1 oder Anspruch 2, **dadurch gekennzeichnet, dass** sie aus einem Gehäuse (1a) besteht, dessen unterer Teil ihres Innenraums einen Flüssigkeitsbehälter (12) bildet, und dessen oberer Teil ihres Innenraums den Luftkompressor (6) und seinen Motor (60) sowie die Zweiphasen-Zerstäuberdüse (5) aufnimmt, wobei das Gehäuse (1a) eine trichterförmige Trennwand (3) aufnimmt, die den oberen Teil vom unteren Teil des Innenvolumens des Gehäuses (1a) trennt.

4. Patrone nach dem vorstehenden Anspruch, **dadurch gekennzeichnet, dass** der Kompressor (6) und sein Motor (60) in einer zylindrischen Hülse (7) angeordnet sind, die sich vertikal im oberen Teil des Innenvolumens des Gehäuses (1a) erstreckt und in Bezug zu Letzterem zentriert ist, wobei die Hülse (7) eine Rückwand (70) aufweist und im oberen Teil durch einen Deckel (72) verschlossen ist, der mit Durchgangsbohrungen (73) für den organisierten Lufteinlass jeweils mit Schikane versehen ist.

5. Patrone nach dem vorstehenden Anspruch, **dadurch gekennzeichnet, dass** die Hülse (7) in Verbindung mit dem Gehäuse (1a) die Expansionskammer (13) des Aerosols abgrenzt, dass das Aerosol in die Expansionskammer (13) durch den unteren Teil in Letztere eintritt, dass die Hülse (7) aus einem wärmeleitenden Material ausgeführt ist, um einen Wärmeaustausch mit dem gebildeten Aerosol zu bewirken, dass die Hülse (7) im oberen Teil einen umlaufenden Flansch (74) zum Verschluss des oberen Teils des Innenvolumens des Gehäuses (1a) und der Expansionskammer (13) aufweist, und dass der Flansch (74) eine Durchgangsbohrung (74b) zur Ableitung des Aerosols aufweist.

6. Patrone nach Anspruch 4 oder Anspruch 5, **dadurch gekennzeichnet, dass** die Hülse (7) an ihrem unteren Teil in eine von der Trennwand (3) getragene Halterung (8) montiert ist, wobei die Halterung mit Durchgangsbohrungen (80) für den Übergang des Aerosols zur Expansionskammer (13) versehen ist, wobei die Hülse (7) innen an ihrer Rückwand (70) eine Aufnahme (70a) aufweist, in der die Zweiphasen-Zerstäuberdüse (5) montiert ist; wobei die Rückwand (70) der Hülse (7) mit einer Durchgangsbohrung versehen ist, die axial mit der Bohrung (55) für die Ableitung des Aerosols aus der Zweiphasen-Zerstäuberdüse (5) ausgerichtet ist; wobei die Düse (5) mit einer Eintrittsbohrung für die Druckluft (53) in Durchgangsverbindung mit dem Druckluftaustritt des Kompressors (6) und zumindest einer Ansaugbohrung (54) für Flüssigkeit in Verbindungsbeziehung vermittels des inneren Saugkanals (4a) der Saugkanüle (4) mit dem Flüssigkeitsbehälter (12) verbunden ist.

7. Patrone nach einem der vorstehenden Ansprüche, **dadurch gekennzeichnet, dass** der im Kompressor (6) enthaltene Druckluftauslass direkt mit dem in der Zweiphasen-Zerstäuberdüse (5) enthaltenen Drucklufteinlass verbunden ist.

8. Patrone nach einem der Ansprüche 1 bis 7, **dadurch gekennzeichnet, dass** die programmierten Veralterungsmittel aus einer elektronischen Schaltung (9) bestehen, die mit dem Motor (60) des Kompressors verbunden ist, um ihn zu steuern und mit elektrischer Energie zu versorgen.

9. Patrone nach dem vorstehenden Anspruch, **dadurch gekennzeichnet, dass** die elektronische Schaltung (9) in der Hülse (7) zwischen dem Motor (60) des Kompressors (6) und dem Deckel (72) montiert ist, dass dieser Deckel (72) Durchgangsbohrungen (72b) zum Durchführen von elektrischen Stiften (93) für die Stromversorgung sowohl der elektronischen Schaltung (9) als auch des Motors (60) aufweist, und dass diese elektrischen Stifte (93) an eine elektrische Energiequelle (94) angeschlossen sind, die sich außerhalb der Patrone befindet.

10. Patrone nach Anspruch 8 oder Anspruch 9, **dadurch gekennzeichnet, dass** die elektronische Schaltung (9) geeignet ist, den Verbrauch von zerstäubter Flüssigkeit zu messen und zu summieren und diesen Verbrauch mit einem Sollwert zu vergleichen, der für die Flüssigkeitsmenge repräsentativ ist, die vor dem ersten Gebrauch im Behälter (12) vorhanden ist, und die elektrische Energieversorgung des Motors (60) des Kompressors (6) zu unterbrechen, wenn der Sollwert erreicht ist.

11. Patrone nach dem vorstehenden Anspruch, **dadurch gekennzeichnet, dass** die elektronische Schaltung (9) zumindest Folgendes integriert:
- einen Speicherblock (90), der eine Angabe bezüglich der Flüssigkeitsmenge enthält, die vor dem ersten Gebrauch des Geräts im Behälter vorhanden ist, eine Angabe bezüglich der Art der Flüssigkeit und eine Angabe bezüglich der Viskosität der zu zerstäubenden Flüssigkeit,
- einen Mikrocontroller (91), der geeignet ist, die momentane Flüssigkeitsverbrauchsrate und die Menge verbrauchter Flüssigkeit zu berechnen, wobei der Mikrocontroller ferner geeignet ist, die verbrauchte Menge mit dem Sollwert zu vergleichen,
- eine Leistungsschnittstelle (92), über die der Motor (60) des Kompressors mit elektrischer Energie versorgt wird, wobei die Leistungsschnittstelle (92) von dem Mikrocontroller (91) gesteuert wird, um die Energieversorgung des Motors (60) zu unterbrechen, wenn der Sollwert erreicht ist.

12. Patrone nach Anspruch 8 oder Anspruch 9, **dadurch gekennzeichnet, dass** die elektronische Schaltung (9) geeignet ist, die Betriebszyklusdauern des Motors (60) zu messen und zu summieren und den erhaltenen Wert mit einem Sollwert zu vergleichen, der für die maximal zulässige Laufzeit des Motors repräsentativ ist, und die elektrische Energieversorgung des Motors (60) zu unterbrechen, wenn der Sollwert erreicht ist, wobei der Sollwert der Zeit entspricht, die für die vollständige Entleerung des Behälters (12) erforderlich ist.

13. Patrone nach dem vorstehenden Anspruch, **dadurch gekennzeichnet, dass** die elektronische Schaltung (9) zumindest Folgendes integriert:
- einen Speicherblock (90), der Daten bezüglich der maximalen Betriebszeit des Kompressormotors enthält,
- einen Mikrocontroller (91), der geeignet ist, die Betriebszyklusdauern des Kompressormotors zu messen und zu summieren und den Wert dieser Messung mit dem Sollwert zu vergleichen,
- eine Leistungsschnittstelle (92), über die der Kompressormotor mit elektrischer Energie versorgt wird, wobei die Leistungsschnittstelle von dem Mikrocontroller gesteuert wird, um die Motorstromversorgung zu unterbrechen, wenn der Sollwert erreicht ist.

14. Aerosol-Diffusionsgerät, **dadurch gekennzeichnet, dass** es eine Patrone nach einem der vorstehenden Ansprüche aufnimmt.

15. Aerosol-Diffusionsgerät, das eine Patrone nach den Ansprüchen 5 und 9 in Kombination aufnimmt, **dadurch gekennzeichnet, dass** es mit einer Düsenöffnung (21) für die Abgabe des Aerosols versehen ist, vor der die Bohrung (74b) positioniert ist, die die Patrone (1) aufweist, und mit einem Gehäuse (2), das mit einer inneren Kammer (20) versehen ist, in der die Patrone entfernbar angeordnet ist, wobei das Gehäuse mit Stiften (93) und mit einer elektrischen Energiequelle (94) ausgestattet ist.

## Claims

1. A cartridge for producing and dispensing an aerosol (1) comprising a container (12) containing a liquid to be sprayed, a two-phase spraying nozzle (5) in communication with the container (12) by an inner channel (4a) of a suction cannula (4) and an air compressor (6) capable of producing a carrier stream of compressed air introduced into the two-phase nozzle (5) so that the contents of the container is first sucked in by Venturi effect, then fractionated and finally propelled first to an expansion chamber (13) and then to the outside, still by the carrier stream, **characterized in that** the elements of which it consists form a non-dismountable assembly and that it includes means of planned obsolescence capable of directly or indirectly determining the total quantity of liquid consumed in previous operating cycles, capable of comparing this value with a setpoint value representative of the quantity of liquid present in the container before the first use, and capable of preventing the subsequent operation of the compressor when the liquid contained in the container (12) has been drained to render said cartridge unusable even if the container of the latter is filled again.

2. The cartridge according to claim 1, **characterized in that** the air compressor (6) is equipped with an electrical actuator motor (60) and **in that** said compressor with its motor are arranged in said cartridge.

3. The cartridge according to claim 1 or claim 2, **characterized in that** it is formed from an enclosure (1a) whereof the lower part of its inner volume forms a liquid container (12) and whereof the upper part of its inner volume receives the air compressor (6) and its motor (60) as well as the two-phase spraying nozzle (5), said enclosure (1a) receiving a partition wall (3) in the shape of a funnel which ensures a separation between the upper part and the lower part of the inner volume of said enclosure (1a).

4. The cartridge according to the preceding claim, **characterized in that** the compressor (6) and its motor (60) are arranged in a cylindrical sheath (7) extending vertically in the upper part of the inner volume of the enclosure (1a) and centered with respect to the latter, said sheath (7) having a bottom wall (70) and being closed in the upper part by a lid (72) provided with through holes (73) for letting in air, organized in a zigzag.

5. The cartridge according to the preceding claim, **characterized in that** the sheath (7) in combination with the enclosure (1a) delimits the expansion chamber (13) of the aerosol, **in that** the aerosol enters the expansion chamber (13) by the lower part of the latter, **in that** the sheath (7) is made of a heat-conducting material in order to effect a heat exchange with the formed aerosol, **in that** the sheath (7), in the upper part, has a peripheral flange (74) for closing the upper part of the inner volume of the enclosure (1a) and of the expansion chamber (13), and **in that** said flange (74) has a through hole (74b) for evacuating the aerosol.

6. The cartridge according to claim 4 or claim 5, **characterized in that** the sheath (7) is mounted by its lower part in a cradle (8) carried by the separating wall (3), said cradle being equipped with through holes (80) for the passage of the aerosol toward the expansion chamber (13), said sheath (7) having on the inside of its bottom wall (70) a casing (70a) wherein the two-phase nozzle (5) is mounted; the bottom wall (70) of the sheath (7) being fitted with a through hole axially aligned with the through hole (55) for evacuating the aerosol from the two-phase nozzle (5); the nozzle (5) being fitted with a through hole (53) for introducing compressed air (53) in communication with the compressed air outlet of the compressor (6) and at least one hole (54) for sucking liquid, in communication by way of the inner suction channel (4a) with the suction cannula (4), with the liquid container (12).

7. The cartridge according to any one of the preceding claims, **characterized in that** the compressed air outlet included in the compressor (6) is directly connected to the compressed air inlet of the two-phase nozzle (5).

8. The cartridge according to any one of claims 1 to 7, **characterized in that** the planned obsolescence means are formed by an electronic circuit (9) connected to the motor (60) of the compressor to control it and supply it with electrical energy.

9. The cartridge according to the preceding claim, **characterized in that** the electronic circuit (9) is mounted in the sheath (7) between the motor (60) of the compressor (6) and the lid (72), that this lid (72) has through holes (72b) for running electrical pins (93) for supplying electrical energy to both the electronic circuit (9) and the motor (60) and that these electrical pins (93) are connected to a source of electrical energy (94) outside said cartridge.

10. The cartridge according to claim 8 or claim 9, **characterized in that** the electronic circuit (9) is capable of measuring and totaling up the consumption of sprayed liquid and comparing this consumption with a setpoint value representative of the quantity of liquid present in the container (12) before the first use, and interrupting the supply of electrical energy to the motor (60) of the compressor (6) when the setpoint value has been reached.

11. The cartridge according to the preceding claim, **characterized in that** the electronic circuit (9) incorporates at least:
- a memory block (90) containing a data item relating to the quantity of liquid present in the container before the first use of the apparatus, a data item relating to the nature of the liquid and a data item relating to the viscosity of the liquid to be sprayed,
- a microcontroller (91) capable of computing the instantaneous flow rate of consumption of liquid, and the quantity of liquid consumed, said microcontroller being in addition capable of comparing the consumed quantity to the setpoint value,
- a power interface (92) via which the motor (60) of the compressor is supplied with electrical energy, said power interface (92) being controlled by the microcontroller (91) to interrupt the powering of the motor (60) when the setpoint value has been reached.

12. The cartridge according to claim 8 or claim 9, **characterized in that** the electronic circuit (9) is capable of measuring and totaling the durations of the operating cycles of the motor (60) and comparing the value obtained to a setpoint value representative of the maximum authorized duration of operation of said motor, and interrupting the supply of electrical energy to said motor (60) when the setpoint value has been reached, this setpoint value corresponding to the time required for complete draining of the container (12).

13. The cartridge according to the preceding claim, **characterized in that** the electronic circuit (9) incorporates at least:
- a memory block (90) containing a data item relating to the maximum duration of use of the compressor motor,
- a microcontroller (91) capable of measuring and totaling the durations of the operating cycles of the compressor motor and comparing the value of this measurement to the setpoint value,
- a power interface (92), via which the compressor motor is supplied with electrical energy, said power interface being controlled by the microcontroller to interrupt the powering of the motor when the setpoint value has been reached.

14. An apparatus for dispensing an aerosol, **characterized in that** it receives a cartridge according to any one of the preceding claims.

15. The apparatus for dispensing an aerosol receiving a cartridge according to claims 5 and 9 taken together, **characterized in that** it is fitted with a jet (21) for delivering the aerosol facing which is positioned the hole (74b) included in the cartridge (1) and a box (1) fitted with an inner chamber (20) wherein the cartridge is removably arranged, said box being equipped with the pins (93) and the electrical energy source (94) .
